# EUROPEAN PATENT SPECIFICATION

(11) **EP 1 707 621 B1**
(45) Date of publication and mention of the grant of the patent: **18.11.2009**
(21) Application number: 06012500.2
(22) Date of filing: 30.06.1998
(51) Int. Cl.: C12N 9/02, C12N 15/53, C12P 21/02

(54) **Mutant bioluminescent protein and process for producing the mutant bioluminescent protein**
Mutiertes Biolumineszenz-Protein und Verfahren zu dessen Herstellung
Protéine mutante bioluminescente et son procédé de fabrication

(30) Priority: 08.07.1997 US 51917 P
(43) Date of publication of application: 04.10.2006
(62) Divisional of application: 98929762.7
(73) Proprietor: KIKKOMAN CORPORATION, Noda-shi, Chiba 278-8601 (JP)
(72) Inventor: Hirokawa, Kozo, c/o Kikkoman Corporation, Noda-shi, Chiba 278-8601 (JP); Kajiyama, Naoki, c/o Kikkoman Corporation, Noda-shi, Chiba 278-8601 (JP); Murakami, Seiji, c/o Kikkoman Corporation, Noda-shi, Chiba 278-8601 (JP)
(74) Representative: Brasnett, Adrian Hugh

(56) References cited:
- EP-A- 0 449 621
- EP-A- 0 524 448
- WO-A-96/22376
- KAJIYAMA N ET AL: "Thermostabilization of firefly luciferase by a single amino acid substitution at position 217." BIOCHEMISTRY. 21 DEC 1993, vol. 32, no. 50, 21 December 1993 (1993-12-21), pages 13795-13799, XP002295272 ISSN: 0006-2960
- WHITE ET AL: "improved thermostability of the north american firefly luciferase: saturation mutagenesis at position 354" BIOCHEMICAL JOURNAL, PORTLAND PRESS, LONDON, GB, vol. 319, 1996, pages 343-350, XP002097112 ISSN: 0264-6021

## Description

The present invention relates to a mutant-type bioluminescent protein and a process for producing the mutant-type bioluminescent protein.

### Background Art

As conventional wild-type firefly luciferases, those derived from Genji firefly (Luciola cruciata), Heike firefly (Luciola lateralis), North American firefly (Photinus pyralis), East European firefly (Luciola mingrelica), Tuchi firefly (Lampyris noctiluca) etc. are known.

Further, mutant-type luciferases (with mutations in thermostability, luminescent color etc.) have also been obtained from these wild-type firefly luciferases as the source.

Improvement of the catalytic efficiency and stability of this enzyme by mutating it is very important. This is because the improvement of catalytic efficiency leads to a reduction in the enzyme used while the improvement of stability makes the enzyme usable under reaction conditions which the wild-type enzyme could not be used.

However, none of such luciferases excellent in stability such as thermostability etc. and with high catalytic efficiency have been reported until now.

WO 96/22376 discloses mutant luciferases from Photinus pyralis, Luciola cruciata and Luciola lateralis comprising substitutions at the amino acid residues 215 and/or 217, 270 and/or 272, and 354 and/or 356.

EP 0 524 448 and Kajiyama et al (Biochemistry 1993, 32, 13795-13799) disclose mutant luciferases of the firefly Luciola cruciata and Luciola lateralis, wherein the threonine residue at position 217 of the amino acid sequence is converted into a hydrophobic amino acid.

Kajiyama et al., Biochemistry (1993) 32(50):13795-13799 describes a Thr217Ile mutant of *Luciola cruciata* which is said to have a higher pH stability and an increased specific activity.

### Disclosure of the Invention

Accordingly, the object of the present invention is to provide a mutant-type luciferases excellent in stability and with high catalytic efficiency. As a result of their eager study to solve the object described above, the present inventors found that mutant-type luciferases with improvements in catalytic efficiency and/or thermostability are obtained by replacement, alternation, removal and addition of at least one amino acid and fusion of a plurality of luciferases.

That is the present invention encompasses:
(1) A bioluminescent protein having the sequence of SEQ ID NO: 5, which has firefly-luciferase activity and comprises luciferases from Genji firefly (Luciola cruciata) and American firefly (Photinus pyralis) fused therein.
(2). A bioluminescent protein having the sequence of SEQ ID NO: 7, which has firefly-luciferase activity and comprises luciferases from Genji firefly (Luciola cruciata) and American firefly (Photinus pyralis) fused therein.
(3). A bioluminescent protein according to (2) which has firefly luciferase activity and has a mutation in an amino acid residue corresponding to the 219-position of the Genji firefly (Luciola cruciata) luciferase.
(4). A bioluminescent protein according to (2) which has firefly luciferase activity and has a mutation in an amino acid residue corresponding to the 239-position of the Genji firefly (Luciola cruciata) luciferase.
(5). A bioluminescent protein according to (2) which has firefly luciferase activity and has a mutation in an amino acid residue corresponding to the 290-position of the Genji firefly (Luciola cruciata) luciferase.
(6). A bioluminescent protein having the sequence of SEQ ID NO: 10 which has firefly luciferase activity and comprises luciferases from Heike firefly (Luciola lateralis) and American firefly (Photinus pyralis) fused therein.
(7). A bioluminescent protein according to (1) to (6) which has improvements in catalytic activity.
(8). A bioluminescent protein according to (1) to (7) wherein the improvements include at least one of 5 kinds of improvements in substrate specificity and maximum reaction rate in respect of catalytic efficiency and thermal stability, pH stability and stability at low ion concentration in respect of stability.

Hereinafter, the present invention is described in detail.

The "luciferase with improvements in pH stability" as used herein refers to the enzyme having any of the following properties: (1) the one with a broadened pH range in which 80 % or more residual activity is maintained, as compared with the conventional luciferase, (2) the one with increased residual activity in a specific pH buffer, as compared with the conventional luciferase, (3) the one with 75 % or more residual activity after treatment in 100 mM acetate buffer (pH 5.5) at 25 °C for 22 hours, and (4) the one with 10 % or more residual activity after treatment in 100 mM CHES buffer (pH 9.0) at 25 °C for 22 hours.

To provide luciferase with improvements in catalytic efficiency or stability by modifying a gene in the present invention, it is necessary to prepare a wild-type luciferase gene and its recombinant DNA.

The wild-type luciferase gene may be any gene derived from beetles (Coleoptera) and includes e.g. genes derived from Genji firefly (Luciola cruciata), Heike firefly (Luciola lateralis), North American firefly (Photinus pyralis), East European firefly (Luciola mingrelica), Tuchi firefly (Lampyris noctiluca) etc.

The wild-type luciferase gene derived from Luciola cruciata and its recombinant DNA can be obtained by a method as described in e.g. Japanese Patent Laid-Open Publication Nos. 34289/1989 and 51086/1989, and the wild-type luciferase gene derived from Luciola lateralis and its recombinant DNA can be obtained by a method described in e.g. Japanese Patent Laid-Open Publication Nos. 13379/1990 and 65780/1990.

Then, the resulting wild-type Coleoptera luciferase gene is modified to give a mutant-type luciferase gene. In this modification, the Coleoptera luciferase gene can be modified as such, or the gene is integrated in vector DNA such as plasmid vector, bacteriophage vector etc. and the resulting recombinant DNA may be modified. By these modified luciferase genes, the mutant-type luciferases, in which at least one amino acid in Coleoptera luciferase has been replaced, altered, removed or added and a plurality of Coleoptera luciferases have been fused therein, are prepared.

First, the replacement, alternation, removal and addition of at least one amino acid in the Coleoptera luciferase can be effected using a wide variety of methods, for example by contacting the Coleoptera luciferase gene or its recombinant DNA with chemicals as mutagen, irradiation with UV light, genetic engineering means or protein engineering means.

The chemicals used as the mutagen in mutagenesis include e.g. hydroxylamine, N-methyl-N'-nitro-N-nitrosoguanidine (NTG), nitrite, sulfite, hydrazine, formic acid, 5-bromouracil etc. The conditions for contacting the chemicals can be varied depending on e.g. the type of chemicals used and are not limited insofar as the desired mutation can be actually induced in the wild-type luciferase gene. Usually, the desired mutation can be induced by contacting the gene with the chemicals preferably at a concentration of 0.5 to 12 M at a reaction temperature of 20 to 80 °C for 10 minutes or more, preferably 10 to 180 minutes. For irradiation with UV light, conventional methods can be followed as described above ("Gendai Kagaku" (Modern Chemistry), pp. 24-30, the June 1989 issue).

As the method of using protein engineering, a means known as site specific mutagenesis can be generally used. Examples are the Kramer method [Kramer, W. et al., Nucl. Acids Res., 12, 9441-9456 (1984); Kramer, W. et al., Methods in Enzymol., 154, 350-367 (1987); Bauer, C. E. et al., Gene, 37, 73-81 (1985)], the Eckstein method [Taylor, J. W. et al., Nucleic Acids Res., 13, 8749-8764 (1985); Taylor, J. W. et al., Nucleic Acids Res. 13, 8765-8785 (1985); Nakamaye, K., et al., Nucleic Acids Res. 14, 9679-9698 (1986)] and the Kunkel method [Kunkel, T. A., Proc. Natl. Acad. Sci., 82, 488-492 (1985); Kunkel, T. A., et al., Methods Enzymol., 154, 367-382 (1987)].

The fusion of a plurality of Coleoptera luciferases can be effected by the following methods: a method which comprises introducing desired restriction enzyme site(s) into one or more luciferase genes by site-specific mutagenesis, then cleaving them with a suitable restriction enzyme and linking the resulting fragments from a plurality of luciferase genes; a method which comprises preparing one or more luciferase gene fragments by polymerase chain reaction with specific primers and then linking them; and the DNA shuffling method [Willem P. C. Stemmer, 370, 389-391 (1994)].

In addition to the gene modification methods described above, organic synthesis or enzyme synthesis methods can be used for direct synthesis of the desired modified luciferase gene. The nucleotide sequence of the desired luciferase gene obtained by the above methods can be determined and confirmed using the chemical modification method of Maxam-Gilbert [Maxam and Gilbert, Methods in Enzymol., 65, 499-560 (1980)], the dideoxynucleotide chain termination method using M13 phage [Messing, et al. Gene, 19, 269-276 (1982)] etc. As a matter of course, the desired luciferase can be obtained using a combination of said mutation methods, that is, by a combination of replacement, alternation, removal and addition of at least one amino acid and fusion of a plurality of Coleoptera luciferases.

By these mutation means, the mutant-type luciferase gene coding for chimera luciferase, i.e. consisting of a plurality of Coleoptera luciferases fused therein, as well as the mutant-type luciferase gene coding for a polypeptide characterized by mutations in amino acid residues corresponding to the 219-, 239- and 290-positions of luciferase from Luciola cruciata and Luciola lateralis, can be obtained. The mutations in amino acids at the 219-, 239- and 290-positions include e.g. those shown in Table 4.

For instance, the 219-, 239- and 290-positions of luciferase from Luciola cruciata and Luciola lateralis corresponding to 217-Val, 237-Ile and 288-Val of luciferase from Photinus pyralis.

The mutant-type luciferase gene obtained in the manner as described above is introduced in a usual manner into vectors such as bacteriophage, cosmid, or plasmid used for transforming prokaryotic or eukaryotic cells, and these vectors can be used to transform or transduce hosts compatible therewith. The hosts herein used include microorganisms belonging to the genus Escherichia, for example E. coli JM101 (ATCC33876), E. coli DH1 (ATCC33849), E. coli HB101 (ATCC33694), E. coli XL1-blue (purchased from Funakoshi K.K.) etc., and if these microorganisms are selected, they are transformed by the Hanahan method (DNA cloning, 1, 109-135 (1985)) etc. or transduced by the method described in Molecular Cloning, pages 256-268, Cold Spring Harbor Laboratory (1982) etc. so that transformed or transduced microorganisms can be obtained.

The resulting strain is screened for a strain having the ability to produce the mutant-type luciferase, whereby the desired transformed or transduced microorganism, i.e. the strain having the ability to produce the mutant-type luciferase by recombinant DNA having the mutant-type luciferase gene inserted into vector DNA, can be obtained. For purification of the novel recombinant DNA from the strain thus obtained, Current Protocols in Molecular Biology (Wiley Interscience, 1989) unit 1.7 etc. can be used. From the recombinant DNA thus obtained, DNA containing the mutant-type luciferase gene can be obtained for example by allowing an restriction enzyme such as EcoRI to act on said plasmid DNA at a temperature of 30 to 40 °C, preferably 37 °C or thereabout, for 1 to 24 hours, preferably 2 hours or thereabout and then subjecting the reaction solution to agarose gel electrophoresis (Molecular Cloning, page 150, Cold Spring Harbor Laboratory (1982)).

Then, the process for producing the mutant-type luciferase of the present invention is described. The mutant-type luciferase of the present invention is obtained by culturing the transformed or transduced microorganism thus obtained and then purifying luciferase from the resulting culture. Although the microorganism may be cultured in a conventional solid medium, it is preferable to employ a liquid medium for culture.

The medium for use in culturing the above strain includes those containing at least one inorganic salts such as sodium chloride, potassium dihydrogen phosphate, dipotassium hydrogen phosphate, magnesium sulfate, magnesium chloride, ferric chloride, ferric sulfate, manganese sulfate etc. added to at least one nitrogen source such as yeast extract, trypton, peptone, meat extract, corn steep liquor, exudate of soybean or wheat bran, etc., and if necessary a suitable amount of sugars (or carbohydrates), vitamins etc. may be added to it.

The initial pH of the medium is preferably adjusted within pH 7 to 9. The microorganism is cultured at 30 to 42 °C, preferably about 37 °C, for 4 to 24 hours, preferably 6 to 20 hours, preferably using submerged aeration culture, shake culture, or stationary culture. For recovery of the mutant-type luciferase from the culture, conventional enzyme purification means can be used. For example, the microorganism is disrupted in a usual manner by ultrasonication or grinding, or the present enzyme is extracted with a lytic enzyme such as lysozyme etc., or the microorganism is autolyzed in the presence of toluene optionally under shaking to release the present enzyme therefrom.

Then, this solution is filtered, centrifuged etc. to remove insolubles, and if necessary, nucleic acid is removed by adding streptomycin sulfate, protamine sulfate, manganese sulfate etc. The solution is then fractionated with ammonium sulfate, alcohol, acetone etc. and the precipitate is recovered whereby a crude enzyme solution is obtained. The crude enzyme solution is subjected to various kinds of chromatography, electrophoresis etc. to give a purified enzyme preparation. For example, methods such as gel filtration using Sephadex, Ultrogel, Bio-Gel etc., adsorption-elution using ion exchangers, electrophoresis using polyacrylamide gel etc., adsorption-elution using hydroxyapatite, sedimentation such as sucrose density-gradient centrifugation etc., affinity chromatography and fractionation using molecular sieve membrane, hollow fiber membrane etc. can be suitably selected or combined to give the purified enzyme preparation.

Whether the purified mutant-type luciferase has the amino acid sequence with the desired mutation or not can be confirmed by conventional amino acid analysis such as automatic amino acid sequencing by the Edman degradation etc.

### Brief Description of the Drawings

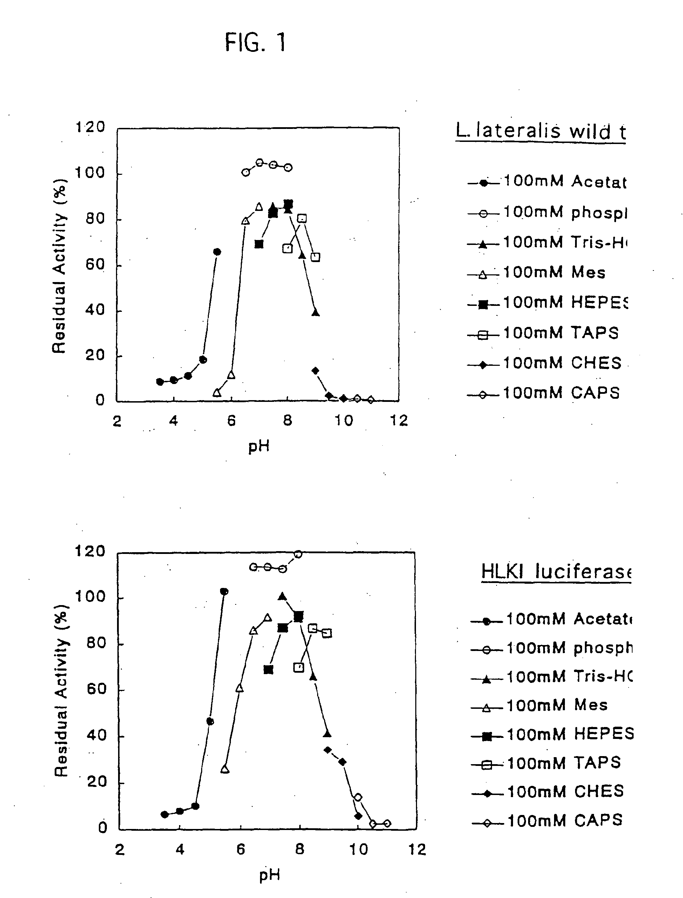

Fig. 1 shows the residual activity of the purified preparation (HLKI luciferase) after treatment in various buffers compared with that of wild-type.

### Best Mode for Carrying Out the Invention

Hereinafter, the present invention is described in more detail by reference to the examples.

### Example 1

10 µg plasmid pT3/T7-LUC (obtained from Clontech) for expression of luciferase derived from an American firefly (Photinus pyralis) was added to 50 µl restriction enzyme buffer K [20 mM Tris-HCl (pH 8.5), 10 mM MgCl₂, 100 mM KCl, 1 mM dithiothreitol] and then cleaved with 20 U each of restriction enzymes SphI and SmaI (Takara Shuzo Co., Ltd.) at 37 °C for 2 hours. This reaction solution was subjected to 0.8 % low-melting agarose gel electrophoresis, and a gel containing an about 1.1-kb DNA fragment containing a C-terminal portion of a luciferase gene derived from Photinus pyralis was cut off and then molten by heating at 65 °C for 5 minutes. To the molten gel was added a 2-fold volume of TE buffer [10 mM Tris-HCl (pH 8.0), 0.5 mM EDTA], and after an equal volume of phenol saturated with TE buffer was added thereto, the mixture was stirred. After centrifugation at 12,000 r.p.m. for 15 minutes, the aqueous layer was recovered and then precipitated with a 2-fold volume of cold ethanol to recover the DNA fragment containing the C-terminal portion of the luciferase gene derived from Photinus pyralis.

Separately, synthetic DNAs (SEQ ID NO:1, CTC TAG CAT GCG AAA ATC TAG; SEQ ID NO:2, CTG CAG GCC TGC AAG CTT GG) [prepared by System 1 Plus DNA synthesizer, Beckman] was added to plasmid pGLf37 (described in Japanese Patent Laid-Open Publication No. 244,942/1993) for expression of luciferase derived from Genji firefly (Luciola cruciata), and polymerase chain reaction (PCR) was carried out as follows. 50 µl PCR reaction solution contained 20 µg plasmid pGLf37, 50 pmol each of the synthetic DNAs, 120 mM Tris-HCl (pH 8.0), 6 mM (NH₄)₂SO₄, 10 mM KCl, 2.5 mM MgSO₄, 0.1 % Triton X-100, 0.001 % BSA, 0.2 mM each of dATP, dGTP, dCTP and dTTP, and 2.5 U of KOD DNA polymerase (Toyobo Co., Ltd.). This mixture was subjected to 25 cycles of PCR, each cycle consisting of incubation at 98 °C for 15 seconds, 65 °C for 2 seconds and 74 °C for 30 seconds in Perkin-Elmer Thermal Cycler PJ2000. To the reaction mixture was added an equal volume of phenol saturated with TE buffer, and the mixture was stirred. After centrifugation at 12,000 r.p.m. for 15 minutes, the aqueous layer was recovered and then precipitated with a 2-fold volume of cold ethanol to recover the DNA fragment. It was dissolved again in TE buffer, then cleaved with SphI and subjected to low-melting agarose gel electrophoresis to recover an about 3.4-kbp DNA fragment containing an N-terminal portion of the luciferase gene derived from Luciola cruciata.

50 ng of the above SphI-SmaI fragment from pT3/T7-LUC and 50 ng of the above SphI-cleaved fragment from pGLf37 were incubated at 15 °C for 16 hours in 20 µl DNA ligase buffer in the presence of 300 U of T4 DNA ligase. The reaction mixture was used to transform E. coli JM109 (Toyobo Co., Ltd.) by the Hana-han method [DNA Cloning, 1, 109-135 (1985)], and ampicillin-resistant colonies were selected.

A plasmid was removed from the formed colonies by the alkali-SDS method, and the structure of the plasmid was confirmed. This plasmid was subjected to reaction with a dye primer Taq sequencing kit (Applied Biosystems) and analyzed by electrophoresis with an ABI 373A DNA sequencer (Applied Biosystems) to determine its nucleotide sequence. The determined nucleotide sequence is shown in SEQ ID NO:6, and the amino acid sequence of a polypeptide translated from said nucleotide sequence is shown in SEQ ID NO:5. The plasmid thus obtained was designated pGAl.

Plasmid pGA1 was used to transform E. coli JM109 in the manner described above to give E. coli JM109 (pGA1). E. coli JM109 (pGA1) was deposited as FERM BP-5990 with the National Institute of Bioscience and Human-Technology, Agency of Industrial Science and Technology, Japan.

E. coli JM109 (pGA1) was inoculated on an LB-amp agar plate [1 % (W/V) Bacto-trypton, 0.5 % (W/V) yeast extract, 0.5 % (W/V) NaCl, (50 µg/ml) ampicillin and 1.4 % (W/V) agar] and cultured at 37 °C. The colony microorganisms appearing 16 hours thereafter were inoculated into 10 ml of an LB-amp medium [1 % (W/V) Bacto-trypton, 0.5 % (W/V) yeast extract, 0.5 % (W/V) NaCl and (50 µ g/ml) ampicillin] and cultured at 37 °C for 18 hours under shaking. This culture, 10 ml, was inoculated into 2 L of the above LB-amp medium and cultured at 30 °C for 6 hours under shaking and then centrifuged at 8,000 r.p.m. for 10 minutes to give 30 g wet microbial pellet. The recovered microorganism was suspended in 20 ml buffer consisting of 0.1 M KH₂PO₄ (pH 7.8), 2 mM EDTA, 1 mM dithiothreitol and 0.2 mg/ml protamine sulfate, and 2 ml of 10 mg/ml lysozyme solution was further added thereto and the mixture was allowed to stand on ice for 15 minutes.

Then, this suspension was frozen in an ethanol/dry ice bath and then allowed to stand at a temperature of 25 °C until it was completely thawed. Further, it was centrifuged at 12,000 r.p.m. for 5 minutes whereby 20 ml crude enzyme was obtained as the supernatant. The crude enzyme solution thus obtained was purified according to the method described in Japanese Patent Laid-Open Publication 141592/1989, and the purified enzyme was designated GA1 luciferase. The Km value of this purified preparation for the substrate ATP was determined. The peak of the emission of light generated by use of the enzyme with the concentration of ATP varying from 0 to 2 mM in a solution containing 50 mM HEPES (pH 7.5), 0.2 mM luciferin and 10 mM MgSO₄ was measured in Luminometer ML3000 (Dynatech) and the result is shown in the table 1 below. The thus determined affinity of the GA1 luciferase for ATP was about 5.73-fold higher than the wild-type Photinus pyralis luciferase and about 11.4-fold higher than the wild-type Luciola cruciata luciferase. This improvement in the affinity of the GA1 luciferase for ATP as compared with that of the wild-type luciferases revealed that the GA1 luciferase is a highly useful enzyme.

**Table 1**

| | Km (mM) |
|---|---|
| Photinus pyralis luciferase | 0.152 |
| Luciola cruciata luciferase | 0.301 |
| GA1 luciferase | 0.0265 |

### Example 2

10 µg plasmid pT3/T7-LUC (obtained from Clontech) for expression of luciferase derived from an American firefly (Photinus pyralis) was added to 50 µl buffer H [50 mM Tris-HCl (pH 7.5), 10 mM MgCl₂, 100 mM NaCl, 1 mM dithiothreitol] and then cleaved with 20 U each of restriction enzymes EcoRV and SalI (Takara Shuzo Co., Ltd.) at 37 °C for 2 hours. This reaction solution was subjected to 0.8 % low-melting agarose gel electrophoresis, and a gel containing an about 0.5-kb DNA fragment containing a C-terminal portion of a luciferase gene derived from Photinus pyralis was cut off and then molten by heating at 65 °C for 5 minutes. To the molten gel was added a 2-fold volume of TE buffer [10 mM Tris-HCl (pH 8.0), 0.5 mM EDTA], and after an equal volume of phenol saturated with TE buffer was added thereto, the mixture was stirred. After centrifugation (12,000 r.p.m. for 15 minutes), the aqueous layer was recovered and then precipitated with a 2-fold volume of cold ethanol to recover a DNA fragment containing a region coding for the C-terminal of luciferase from Photinus pyralis.

Separately, synthetic DNAs (SEQ ID NO:3. ATC CTT TGT ATT TGA TTA AAG; SEQ ID NO:4, TCT AGA GTC GAC CTG CAG GC) [prepared by System 1 Plus DNA synthesizer, Beckman] was added to plasmid pGLf37 T-M-2 (described in Japanese Patent Laid-Open Publication No. 244,942/1993) for expression of thermostable luciferase derived from Genji firefly (Luciola cruciata), and polymerase chain reaction (PCR) was carried out as follows. 50 µl PCR reaction solution contained 20 µg plasmid pGLf37 T-M-2, 50 pmol each of the synthetic DNAs, 120 mM Tris-HCl (pH 8.0), 6 mM (NH₄)₂SO₄, 10 mM KCl, 2.5 mM MgSO₄, 0.1 % Triton X-100, 0.001 % BSA, 0.2 mM each of dATP, dGTP, dCTP and dTTP, and 2.5 U of KOD DNA polymerase (Toyobo Co., Ltd.). This mixture was subjected to 25 cycles of PCR, each cycle consisting of incubation at 98 °C for 15 seconds, 65 °C for 2 seconds and 74 °C for 30 seconds in Perkin-Elmer Thermal Cycler PJ2000. To the reaction mixture was added an equal volume of phenol saturated with TE buffer, and the mixture was stirred. After centrifugation (12,000 r.p.m. for 15 minutes), the aqueous layer was recovered and then precipitated with a 2-fold volume of cold ethanol to recover the DNA fragment. It was dissolved again in TE buffer, then cleaved with SalI and subjected to low-melting agarose gel electrophoresis to recover a DNA fragment containing a region coding for the N-terminal of luciferase derived from Luciola cruciata. In this region, a thermostable mutation (Thr217Ile) derived from pGLf37 T-M-2 was contained.

50 ng of the above about 0.5-kbp EcoRV-SalI fragment derived from pT3/T7-LUC and 50 ng of the above about 4-kbp SalI-cleaved fragment derived from pGLf37 T-M-2 were incubated at 15 °C for 16 hours in 20 µl DNA ligase buffer in the presence of 300 U of T4 DNA ligase. The reaction mixture was used to transform E. coli JM109 (Toyobo Co., Ltd.) by the Hana-han method [DNA Cloning, 1, 109-135 (1985)], and ampicillin-resistant colonies were selected.

A plasmid was removed from the formed colonies by the alkali-SDS method, This plasmid was subjected to reaction with a dye primer Taq sequencing kit (Applied Biosystems) and analyzed by electrophoresis with an ABI 373A DNA sequencer (Applied Biosystems) to determine its nucleotide sequence. The determined nucleotide sequence is shown in SEQ ID NO:8, and the amino acid sequence of a polypeptide translated from said nucleotide sequence is shown in SEQ ID NO:7. The plasmid thus obtained was designated pGGA1.

Plasmid pGGA1 was used to transform E. coli JM109 in the manner described above to give E. coli JM109 (pGGA1). E. coli JM109 (pGGA1) was deposited as FERM BP-5989 with the National Institute of Bioscience and Human-Technology, Agency of Industrial Science and Technology, Japan.

E. coli JM109 (pGGA1) was inoculated on an LB-amp agar plate [1 % (W/V) Bacto-trypton, 0.5 % (W/V) yeast extract, 0.5 % (W/V) NaCl, (50 µg/ml) ampicillin and 1.4 % (W/V) agar] and cultured at 37 °C. The colonies appearing 16 hours thereafter were cultured at 37 °C for 18 hours under shaking in 10 ml of an LB-amp medium [1 % (W/V) Bacto-trypton, 0.5 % (W/V) yeast extract, 0.5 % (W/V) NaCl and (50 µg/ml) ampicillin]. This culture, 10 ml, was inoculated into 2 L of the above LB-amp medium and cultured at 30 °C for 6 hours under shaking and then centrifuged at 8000 r.p.m. for 10 minutes to give 30 g wet microbial pellet. The recovered microorganism was suspended in 20 ml buffer consisting of 0.1 M KH₂PO₄ (pH 7.8), 2 mM EDTA, 1 mM dithiothreitol and 0.2 mg/ml protamine sulfate, and 2 ml of 10 mg/ml lysozyme solution was further added thereto and the mixture was allowed to stand on ice for 15 minutes. Then, this suspension was frozen in an ethanol/dry ice bath and then allowed to stand at a temperature of 25 °C until it was completely thawed. Further, it was centrifuged at 12,000 r.p.m. for 5 minutes whereby 20 ml crude enzyme was obtained as the supernatant. The crude enzyme solution thus obtained was purified according to the method described in Japanese Patent Laid-Open Publication 141592/1989, and the purified enzyme was designated GGA1 luciferase.

The Km value of this purified GGA1 enzyme for the substrate ATP was determined (Table 2). The result indicated that the affinity of the GGA1 luciferase for ATP was about 1.46 fold higher than the wild-type Photinus pyralis luciferase and about 2.89-fold higher than the wild-type Luciola cruciata luciferase. This improvement in the affinity of the GGA1 luciferase for ATP as compared with that of the wild-type luciferase reveals that the GGA1 luciferases is a very useful enzyme.

**Table 2**

| | Km (mM) |
|---|---|
| Photinus pyralis luciferase | 0.152 |
| Luciola cruciata luciferase | 0.301 |
| GGA1 luciferase | 0.104 |

This purified enzyme was examined for thermal stability where the remaining activity after treated at 50 °C in 0.05 M potassium phosphate buffer (pH 7.8) with 10 % ammonium sulfate saturation was determined. The result indicated that this enzyme maintained 80 % or more of the original activity even after treatment at 50 °C for 20 minutes, and it was thus found that the thermal stability of this enzyme has been improved as compared with that of the wild-type Photinus pyralis luciferase and the thermostable Luciola cruciata luciferase.

### Example 3

10 µg plasmid pT3/T7-LUC (obtained from Clontech) for expression of luciferase derived from an American firefly (Photinus pyralis) was added to 50 µl restriction enzyme buffer H [50 mM Tris-HCl (pH 7.5), 10 mM MgCl₂, 100 mM NaCl, 1 mM dithiothreitol] and then cleaved with 20 U of restriction enzyme EcoRV (Takara Shuzo Co., Ltd.) at 37 °C for 2 hours. This reaction solution was subjected to 0.8 % low-melting agarose gel electrophoresis, and a gel containing an about 500 bp DNA fragment containing a region coding for the C-terminal of the luciferase from Photinus pyralis was cut off and then molten by heating at 65 °C for 5 minutes. To the molten gel was added a 2-fold volume of TE buffer [10 mM Tris-HCl (pH 8.0), 0.5 mM EDTA], and after an equal volume of phenol saturated with TE buffer was added thereto, the mixture was stirred. After centrifugation (12,000 r.p.m. for 15 minutes), the aqueous layer was recovered and then precipitated with a 2-fold volume of cold ethanol to recover the DNA fragment containing a region coding for the C-terminal of the Photinus pyralis luciferase

Separately, 10 µg plasmid pHLf7-217Leu (Japanese Patent Application Laid-Open Publication No. 244942/93) for expression of luciferase derived from Heike firefly (Luciola lateralis) was added to 50 µl restriction enzyme buffer T [33 mM Tris-HCl (pH 7.9), 10 mM magnesium acetate, 66 mM potassium acetate, 0.5 mM dithiothreitol] and then cleaved with 20 U each of restriction enzymes EcoRV and NaeI (Takara Shuzo Co., Ltd.) at 37 °C for 2 hours. This reaction solution was subjected to 0.8 % low-melting agarose gel electrophoresis, and a gel containing an about 4.3 kbp DNA fragment containing the N-terminal of the luciferase from Luciola lateralis was cut off and then molten by heating at 65 °C for 5 minutes. To the molten gel was added a 2-fold volume of TE buffer [10 mM Tris-HCl (pH 8.0), 0.5 mM EDTA], and after an equal volume of phenol saturated with TE buffer was added thereto, the mixture was stirred. After centrifugation (12,000 r.p.m. for 15 minutes), the aqueous layer was recovered and then precipitated with a 2-fold volume of cold ethanol to recover the DNA fragment containing a region coding for the N-terminal of the Luciola lateralis luciferase.

50 ng of the above EcoRV-EcoRV fragment from pT3/T7-LUC and 50 ng of the above EcoRV-NaeI fragment from pHLf7-217Leu were incubated at 15 °C for 16 hours in 20 µl DNA ligase buffer in the presence of 300 U of T4 DNA ligase. The reaction mixture was used to transform E. coli JM 109 by the Hana-han method [DNA Cloning, 1, 109-135 (1985)], and ampicillin-resistant colonies were selected. A crude enzyme was prepared from the selected colonies in the method described in Example 1, and a plasmid was removed by the alkali-SDS method from those having luminescence activity, and the structure of the plasmid was confirmed. This plasmid was subjected to reaction with a dye primer Taq sequencing kit (Applied Biosystems) and analyzed by electrophoresis with an ABI 373A DNA sequencer (Applied Biosystems) to determine its nucleotide sequence (SEQ ID NO:9). The amino acid sequence of a polypeptide estimated to be translated from said nucleotide sequence is shown in SEQ ID NO:10. The plasmid thus obtained was designated pHHA1.

Plasmid pHHA1 was used to transform E. coli JM 109 in the above method to give E. coli JM 109 (pHHA1). The E. coli JM 109 (pHHA1) has been deposited as FERM BP-6203 with the National Institute of Bioscience and Human-Technology, Agency of Industrial Science and Technology, Japan.

Further, its crude enzyme solution was prepared using the method described in Example 1 and the enzyme was purified in a method described in Japanese Patent Application Laid-Open Publication No. 141592/89. The purified enzyme was designated HHA1 luciferase. The affinity of HHA1 luciferase for the substrate ATP was determined. The peak of the emission of light generated by use of the enzyme with the concentration of ATP varying from 0 to 2 mM in a solution containing 50 mM Tricine buffer (pH 7.8), 0.2 mM luciferin and 10 mM MgSO₄ was measured in Luminometer ML3000 (Dynatech). The affinity (Km value) of the HHA1 luciferase for ATP was thus determined (Table 3). The affinity of the HHA1 luciferase for ATP was improved as compared with those of the Photinus pyralis and Luciola lateralis luciferases, indicating that the HHA1 luciferase is a highly useful enzyme.

**Table 3**

| | Km (mM) |
|---|---|
| Photinus pyralis luciferase | 0.161 |
| Luciola lateralis luciferase | 0.197 |
| HHA1 luciferase | 0.123 |

### Example 4

To introduce an arbitrary mutation into the luciferase gene, plasmid pGGA1 described in Example 2 was treated at 65 °C for 2 hours in 0.1 M sodium phosphate buffer (pH 6.0) containing 0.8 M hydroxylamine and 1 mM EDTA according to the method of Kironde et al. [Biochem. J., 259, 421-426 (1989)]. The plasmid thus subjected to mutagenesis was desalted by passing it through a G60 DNA grade Nick column (Pharmacia), and then E. coli JM 109 was transformed with this plasmid.

The resulting transformant was inoculated on an LB-amp plate [1.0 % (W/V) Bacto-trypton, 0.5 % (W/V) yeast extract, 0.5 % (W/V) NaCl, 1.5 % (W/V) agar and 50 µg/ml ampicillin] and cultured at 37 °C for 12 hours. The resulting colonies were transferred onto a nitrocellulose filter, and said filter was immersed in 0.1 M sodium citrate buffer (pH 5.0) containing 0.5 mM luciferin [Wood & DeLuca, Anal. Biochem., 161, 501-507 (1987)]. The emission of light from said colonies was monitored, and 3 strains with raised emission could be obtained. These strains were designated E. coli JM 109 (pGGA2-1), E. coli JM 109 (pGGA1-4) and E. coli JM 109 (pGGA2-4), respectively. The E. coli JM 109 (pGGA2-1)., E. coli JM 109 (pGGA1-4) and E. coli JM 109 (pGGA2-4) thus obtained have been deposited respectively as FERM BP-6206, FERM BP-6205 and FERM BP-6204 with the National Institute of Bioscience and Human-Technology, Agency of Industrial Science and Technology. Plasmids were removed from these strains by alkali-SDS method. These plasmids were subjected to reaction with a dye primer Taq sequencing kit (Applied Biosystems) and analyzed by electrophoresis with an ABI 373A DNA sequencer (Applied Biosystems) to determine their mutation sites (Table 4).

**Table 4**

| | Position and Nucleotide Change | Position and Amino Acid Change |
|---|---|---|
| E. coli JM 109 (pGGA2-1) | 656-position C → T | 219-position Thr → Ile |
| E. coli JM 109 (pGGA1-4) | 868-position G → A | 290-position Val → Ile |
| E. coli JM 109 (pGGA2-4) | 715-position G → A | 239-position Val → Ile |

From the E. coli JM 109 (pGGA2-1), E. coli JM 109 (pGGA1-4), E. coli JM 109 (pGGA2-4), their crude enzyme solutions were extracted by the method described in Example 1, and these mutant enzymes were purified in the method described in Japanese Patent Laid-Open Publication No. 141592/1989. The purified enzymes were designated GGA1 T219I luciferase, GGA1 V290I luciferase and GGA1 V239I luciferase, respectively. These enzymes were determined for their catalyst efficiency (Vmax/Km) toward the substrate ATP. The luminescent reaction was carried out by mixing, with each enzyme, a substrate mixture containing ATP at concentrations varying from 0 to 1.0×10⁻³ mM in 50 mM Tricine buffer (pH 7.8), 2.0 mM luciferin and 10 mM MgSO₄. The emission of light from 5 seconds to 15 seconds after initiation of the reaction was integrated in Luminometer ML3000 (Dynatech) to determine catalytic efficiency (Vmax/Km). As shown in the table below, it was confirmed that as compared with the GGA1 luciferase, catalytic efficiency was improved by mutating each of the amino acids at the 219-, 290-, and 239-positions.

**Table 5**

| | Vmax/Km (×10⁹ RLU/mg) |
|---|---|
| GGA1 luciferase | 1.22 |
| GGA1 T219I luciferase | 2.16 |
| GGA1 V290I luciferase | 1.70 |
| GGA1 V239I luciferase | 1.58 |

### Example 5

To obtain mutant luciferase with improvements in pH stability, an arbitrary mutation was introduced to the wild-type luciferase gene.

For mutation, PCR was conducted in the presence of 0.5 mM Mn²⁺ reported to cause frequent mutations (A Journal of Methods in Cell and Molecular Biology, Vol. 1, No. 1 (1989), pp. 11-15) where plasmid pHLf7 for expression of luciferase derived from Heike firefly (Luciola lateralis) (described in Japanese Patent Application Laid-Open Publication No. 171189/90) was used as a template and oligonucleotides shown in SEQ ID NOS: 11 (AGAGATCCAA TTTATGGAAA C) and 12 (AGCGTGAGAA AATCTGATCA C) were used as primers. After reaction, the reaction solution was precipitated with a 2-fold volume of cold ethanol. The resulting DNA was dissolved again in TE buffer, 10 U of T4 polynucleotide kinase (Takara Shuzo Co., Ltd.) in T4 polynucleotide kinase buffer was then added, and the mixture was reacted at 37 °C for 30 minutes. This reaction solution was then subjected to 0.8 % low-melting agarose gel electrophoresis, and a gel containing an about 5 kbp DNA fragment was cut off and then molten by heating at 65 °C for 5 minutes. To the molten gel was added a 2-fold volume of TE buffer [10 mM Tris-HCl (pH 8.0), 0.5 mM EDTA], and after an equal volume of phenol saturated with TE buffer was added thereto, the mixture was stirred. After centrifugation (12,000 r.p.m. for 15 minutes), the aqueous layer was recovered and then precipitated with a 2-fold volume of cold ethanol to recover the about 5 kbp DNA fragment. 50 ng of the about 5 kbp DNA fragment thus recovered was incubated at 15 °C for 16 hours in 20 µl DNA ligase buffer in the presence of 10 U of T4 DNA ligase (Toyobo). The reaction mixture was used to transform E. coli JM 109 by the Hana-han method [DNA Cloning, 1, 109-135 (1985)], and ampicillin-resistant colonies were selected on an LB+Amp plate [1.0 % (W/V) Bacto-trypton, 0.5 % (W/V) yeast extract, 0.5 % (W/V) NaCl, 1.4 % (W/V) Bacto-agar and 50 µg/ml ampicillin].

The resulting colonies were cultured in LB medium, and their crude enzyme was prepared by the method described in Example 1. The crude enzyme was treated in 100 mM acetate buffer (pH 5.5) at 25 °C for 22 hours to screen a strain with the activity not lowered. As a result, in contrast to the wild-type losing the activity to a level of about 70 % or less, a strain hardly losing the activity was obtained. A plasmid was removed from this strain by the alkali-SDS method, subjected to reaction with a dye primer Taq sequencing kit (Applied Biosystems), and analyzed by electrophoresis with an ABI 373A DNA sequencer (Applied Biosystems) to determine its nucleotide sequence (SEQ ID NO:13). The amino acid sequence deduced from this nucleotide sequence is shown in SEQ ID NO:14. The plasmid thus obtained was designated pHLKI.

Plasmid pHLKI was used to transform E. coli JM 109 in the method described above. The resulting E. coli JM 109 (pHLKI) has been deposited as FERM BP-6347 with the National Institute of Bioscience and Human-Technology, Agency of Industrial Science and Technology, Japan.

It was cultured in LB medium, its crude enzyme solution was then prepared by the method described in Example 1 and the enzyme was purified in a method described in Japanese Patent Application Laid-Open Publication No. 141592/89. This purified preparation (HLKI luciferase) was treated in various buffers at 25 °C for 22 hours and measured for its residual activity (Fig. 1). As can be seen from Fig. 1, the resulting HLKI luciferase exhibits higher residual activity in the broad range of pH 5.0 to 10.0 than that of the wild strain. As a specific example, its residual activity in the ranges pH 5.0 to 6.0 and pH 9.0 to 10.0 is shown in Table 6.

In Table 6, HLKI luciferase exhibited 2.5-fold or more higher residual activity in 100 mM acetate buffer, pH 5.0 in the acid range than the Luciola lateralis wild-type counterpart. Further, it exhibited 6-fold or more higher residual activity in 100 mM Mes buffer, pH 5.5. In the alkali range, HLKI luciferase exhibited 2.5-fold or more higher residual activity in 100 mM CHES buffer, pH 9.0 and 13-fold or more higher residual activity in 100 mM CHES buffer, pH 9.5 respectively than that of the Luciola lateralis wild-type counterpart. This comparison between HLKI luciferase and the Luciola lateralis wild-type counterpart in the residual activity in buffers at specific pH values indicates that the residual activity is increased in HLKI luciferase.

It is further understood that HLKI luciferase maintains 80 % or more residual activity in the range from pH 6.0-6.5 (100 mM Mes buffer) to pH 9.0 (100 mM TAPS buffer), as opposed to the Luciola lateralis wild-type counterpart which maintains 80 % or more residual activity in the range of pH 6.5 (100 mM Mes buffer) to pH 8.5 (100 mM TAPS buffer). This indicates that the pH range in which HLKI luciferase maintains 80 % or more residual activity is broader than that of the Luciola lateralis wild-type counterpart.

These results indicate that HLKI luciferase has higher pH stability than the enzyme of the wild strain, and according to this property, it can also be reacted in the pH range not applicable to the enzyme of the wild-type counterpart, so it is extremely useful.

**Table 6**

| | Acid range | | | | Alkali range | | |
|---|---|---|---|---|---|---|---|
| | Acetate buffer | | Mes buffer | | CHES buffer | | CAPS buffer |
| pH | 5.0 | 5.5 | 5.5 | 6.0 | 9.0 | 9.5 | 10.0 |
| L. lateralis wild type | 18.0 | 66.0 | 3.90 | 12.0 | 13.3 | 2.10 | 0.800 |
| HLKI luciferase | 46.0 | 102 | 26.3 | 61.0 | 33.9 | 28.8 | 13.7 |

### Effect of the Invention

According to the present invention, there can be provided luciferase excellent in thermostability etc. and with high catalytic efficiency.

### SEQUENCE LISTING

SEQ ID NO: 1
   SEQUENCE LENGTH: 21
   SEQUENCE TYPE: nucleic acid
   STRANDEDNESS: single
   TOPOLOGY: linear
   MOLECULE TYPE: Other nucleic acid
   SEQUENCE DESCRIPTION:
   CTC TAG CAT GCG AAA ATC TAG
SEQ ID NO: 2
   SEQUENCE LENGTH: 20
   SEQUENCE TYPE: nucleic acid
   STRANDEDNESS: single
   TOPOLOGY: linear
   MOLECULE TYPE: Other nucleic acid
   SEQUENCE DESCRIPTION:
   CTG CAG GCC TGC AAG CTT GG
SEQ ID NO: 3
   SEQUENCE LENGTH: 21
   SEQUENCE TYPE: nucleic acid
   STRANDEDNESS: single
   TOPOLOGY: linear
   MOLECULE TYPE: Other nucleic acid
   SEQUENCE DESCRIPTION:
   ATC CTT TGT ATT TGA TTA AAG
SEQ ID NO: 4
   SEQUENCE LENGTH: 20
   SEQUENCE TYPE: nucleic acid
   STRANDEDNESS: single
   TOPOLOGY: linear
   MOLECULE TYPE: Other nucleic acid
   SEQUENCE DESCRIPTION:
   TCT AGA GTC GAC CTG CAG GC
SEQ ID NO: 5
   SEQUENCE LENGTH: 552
   SEQUENCE TYPE: amino acid
   TOPOLOGY: linear
   MOLECULE TYPE: peptide
   ORIGINAL SOURCE: Luciola cruciata and Photinus pyralis
   SEQUENCE DESCRIPTION: 
Met Glu Asn Met Glu Asn Asp Glu Asn Ile Val Val Gly Pro Lys
1               5                   10                  15
Pro Phe Tyr Pro Ile Glu Glu Gly Ser Ala Gly Thr Gln Leu Arg
                20                  25                  30
Lys Tyr Met Glu Arg Tyr Ala Lys Leu Gly Ala Ile Ala Phe Thr
                35                  40                  45
Asn Ala Val Thr Gly Val Asp Tyr Ser Tyr Ala Glu Tyr Leu Glu
                50                  55                  60
Lys Ser Cys Cys Leu Gly Lys Ala Leu Gln Asn Tyr Gly Leu Val
                65                  70                  75
Val Asp Gly Arg Ile Ala Leu Cys Ser Glu Asn Cys Glu Glu Phe
                80                  85                  90
Phe Ile Pro Val Ile Ala Gly Leu Phe Ile Gly Val Gly Val Ala
 
                95                  100                 105
Pro Thr Asn Glu Ile Tyr Thr Leu Arg Glu Leu Val His Ser Leu
                110                 115                 120
Gly Ile Ser Lys Pro Thr Ile Val Phe Ser Ser Lys Lys Gly Leu
                125                 130                 135
Asp Lys Val Ile Thr Val Gln Lys Thr Val Thr Thr Ile Lys Thr
                140                 145                 150
Ile Val Ile Leu Asp Ser Lys Val Asp Tyr Arg Gly Tyr Gln Cys
                155                 160                 165
Leu Asp Thr Phe Ile Lys Arg Asn Thr Pro Pro Gly Phe Gln Ala
                170                 175                 180
Ser Ser Phe Lys Thr Val Glu Val Asp Arg Lys Glu Gln Val Ala
                185                 190                 195
Leu Ile Met Asn Ser Ser Gly Ser Thr Gly Leu Pro Lys Gly Val
                200                 205                 210
Gln Leu Thr His Glu Asn Thr Val Thr Arg Phe Ser His Ala Arg
                215                 220                 225
Asp Pro Ile Phe Gly Asn Gln Ile Ile Pro Asp Thr Ala Ile Leu
                230                 235                 240
Ser Val Val Pro Phe His His Gly Phe Gly Met Phe Thr Thr Leu
                245                 250                 255
Gly Tyr Leu Ile Cys Gly Phe Arg Val Val Leu Met Tyr Arg Phe
                260                 265                 270
Glu Glu Glu Leu Phe Leu Arg Ser Leu Gln Asp Tyr Lys Ile Gln
                275                 280                 285
Ser Ala Leu Leu Val Pro Thr Leu Phe Ser Phe Phe Ala Lys Ser
                290                 295                 300
Thr Leu Ile Asp Lys Tyr Asp Leu Ser Asn Leu His Glu Ile Ala
                305                 310                 315
 
Ser Gly Gly Ala Pro Leu Ser Lys Glu Val Gly Glu Ala Val Ala
                320                 325                 330
Lys Arg Phe His Leu Pro Gly Ile Arg Gln Gly Tyr Gly Leu Thr
                335                 340                 345
Glu Thr Thr Ser Ala Ile Leu Ile Thr Pro Glu Gly Asp Asp Lys
                350                 355                 360
Pro Gly Ala Val Gly Lys Val Val Pro Phe Phe Glu Ala Lys Val
                365                 370                 375
Val Asp Leu Asp Thr Gly Lys Thr Leu Gly Val Asn Gln Arg Gly
                380                 385                 390
Glu Leu Cys Val Arg Gly Pro Met Ile Met Ser Gly Tyr Val Asn
                395                 400                 405
Asn Pro Glu Ala Thr Asn Ala Leu Ile Asp Lys Asp Gly Trp Leu
                410                 415                 420
His Ser Gly Asp Ile Ala Tyr Trp Asp Glu Asp Glu His Phe Phe
                425                 430                 435
Ile Val Asp Arg Leu Lys Ser Leu Ile Lys Tyr Lys Gly Tyr Gln
                440                 445                 450
Val Ala Pro Ala Glu Leu Glu Ser Ile Leu Leu Gln His Pro Asn
                455                 460                 465
Ile Phe Asp Ala Gly Val Ala Gly Leu Pro Asp Asp Asp Ala Gly
                470                 475                 480
Glu Leu Pro Ala Ala Val Val Val Leu Glu His Gly Lys Thr Met
                485                 490                 495
Thr Glu Lys Glu Ile Val Asp Tyr Val Ala Ser Gln Val Thr Thr
                500                 505                 510
Ala Lys Lys Leu Arg Gly Gly Val Val Phe Val Asp Glu Val Pro
                515                 520                 525
Lys Gly Leu Thr Gly Lys Leu Asp Ala Arg Lys Ile Arg Glu Ile
 
                530                 535                 540
Leu Ile Lys Ala Lys Lys Gly Gly Lys Ser Lys Leu
545 550 552
SEQ ID NO: 6
   SEQUENCE LENGTH: 1656
   SEQUENCE TYPE: nucleic acid
   STRANDEDNESS: single
   TOPOLOGY: linear
   MOLECULE TYPE: cDNA to mRNA
   ORIGINAL SOURCE: Luciola cruciata and Photinus pyralis
   SEQUENCE DESCRIPTION: 
ATG GAA AAC ATG GAA AAC GAT GAA AAT ATT GTA GTT GGA CCT AAA CCG 48
TTT TAC CCT ATC GAA GAG GGA TCT GCT GGA ACA CAA TTA CGC AAA TAC 96
ATG GAG CGA TAT GCA AAA CTT GGC GCA ATT GCT TTT ACA AAT GCA GTT 144
ACT GGT GTT GAT TAT TCT TAC GCC GAA TAC TTG GAG AAA TCA TGT TGT 192
CTA GGA AAA GCT TTG CAA AAT TAT GGT TTG GTT GTT GAT GGC AGA ATT 240
GCG TTA TGC AGT GAA AAC TGT GAA GAA TTT TTT ATT CCT GTA ATA GCC 288
GGA CTG TTT ATA GGT GTA GGT GTT GCA CCC ACT AAT GAG ATT TAC ACT 336
TTA CGT GAA CTG GTT CAC AGT TTA GGT ATC TCT AAA CCA ACA ATT GTA 384
TTT AGT TCT AAA AAA GGC TTA GAT AAA GTT ATA ACA GTA CAG AAA ACA 432
GTA ACT ACT ATT AAA ACC ATT GTT ATA CTA GAT AGC AAA GTT GAT TAT 480
CGA GGA TAT CAA TGT CTG GAC ACC TTT ATA AAA AGA AAC ACT CCA CCA 528
GGT TTT CAA GCA TCC AGT TTC AAA ACT GTG GAA GTT GAC CGT AAA GAA 576
CAA GTT GCT CTT ATA ATG AAC TCT TCG GGT TCT ACC GGT TTG CCA AAA 624
GGC GTA CAA CTT ACT CAC GAA AAT ACA GTC ACT AGA TTT TCG CAT GCC 672
AGA GAT CCT ATT TTT GGC AAT CAA ATC ATT CCG GAT ACT GCG ATT TTA 720
AGT GTT GTT CCA TTC CAT CAC GGT TTT GGA ATG TTT ACT ACA CTC GGA 768
TAT TTG ATA TGT GGA TTT CGA GTC GTC TTA ATG TAT AGA TTT GAA GAA 816
 
GAG CTG TTT TTA CGA TCC CTT CAG GAT TAC AAA ATT CAA AGT GCG TTG 864
CTA GTA CCA ACC CTA TTT TCA TTC TTC GCC AAA AGC ACT CTG ATT GAC 912
AAA TAC GAT TTA TCT AAT TTA CAC GAA ATT GCT TCT GGG GGC GCA CCT 960
CTT TCG AAA GAA GTC GGG GAA GCG GTT GCA AAA CGC TTC CAT CTT CCA 1008
GGG ATA CGA CAA GGA TAT GGG CTC ACT GAG ACT ACA TCA GCT ATT CTG 1056
ATT ACA CCC GAG GGG GAT GAT AAA CCG GGC GCG GTC GGT AAA GTT GTT 1104
CCA TTT TTT GAA GCG AAG GTT GTG GAT CTG GAT ACC GGG AAA ACG CTG 1152
GGC GTT AAT CAG AGA GGC GAA TTA TGT GTC AGA GGA CCT ATG ATT ATG 1200
TCC GGT TAT GTA AAC AAT CCG GAA GCG ACC AAC GCC TTG ATT GAC AAG 1248
GAT GGA TGG CTA CAT TCT GGA GAC ATA GCT TAC TGG GAC GAA GAC GAA 1296
CAC TTC TTC ATA GTT GAC CGC TTG AAG TCT TTA ATT AAA TAC AAA GGA 1344
TAT CAG GTG GCC CCC GCT GAA TTG GAA TCG ATA TTG TTA CAA CAC CCC 1392
AAC ATC TTC GAC GCG GGC GTG GCA GGT CTT CCC GAC GAT GAC GCC GGT 1440
GAA CTT CCC GCC GCC GTT GTT GTT TTG GAG CAC GGA AAG ACG ATG ACG 1488
GAA AAA GAG ATC GTG GAT TAC GTC GCC AGT CAA GTA ACA ACC GCG AAA 1536
AAG TTG CGC GGA GGA GTT GTG TTT GTG GAC GAA GTA CCG AAA GGT CTT 1584
ACC GGA AAA CTC GAC GCA AGA AAA ATC AGA GAG ATC CTC ATA AAG GCC 1632
AAG AAG GGC GGA AAG TCC AAA TTG 1656
SEQ ID NO: 7
   SEQUENCE LENGTH: 552
   SEQUENCE TYPE: amino acid
   TOPOLOGY: linear
   MOLECULE TYPE: peptide
   ORIGINAL SOURCE: Luciola cruciata and Photinus pyralis
   SEQUENCE DESCRIPTION: 
Met Glu Asn Met Glu Asn Asp Glu Asn Ile Val Val Gly Pro Lys
1               5                   10                  15
Pro Phe Tyr Pro Ile Glu Glu Gly Ser Ala Gly Thr Gln Leu Arg
 
                20                  25                  30
Lys Tyr Met Glu Arg Tyr Ala Lys Leu Gly Ala Ile Ala Phe Thr
                35                  40                  45
Asn Ala Val Thr Gly Val Asp Tyr Ser Tyr Ala Glu Tyr Leu Glu
                50                  55                  60
Lys Ser Cys Cys Leu Gly Lys Ala Leu Gln Asn Tyr Gly Leu Val
                65                  70                  75
Val Asp Gly Arg Ile Ala Leu Cys Ser Glu Asn Cys Glu Glu Phe
                80                  85                  90
Phe Ile Pro Val Ile Ala Gly Leu Phe Ile Gly Val Gly Val Ala
                95                  100                 105
Pro Thr Asn Glu Ile Tyr Thr Leu Arg Glu Leu Val His Ser Leu
                110                 115                 120
Gly Ile Ser Lys Pro Thr Ile Val Phe Ser Ser Lys Lys Gly Leu
                125                 130                 135
Asp Lys Val Ile Thr Val Gln Lys Thr Val Thr Thr Ile Lys Thr
                140                 145                 150
Ile Val Ile Leu Asp Ser Lys Val Asp Tyr Arg Gly Tyr Gln Cys
                155                 160                 165
Leu Asp Thr Phe Ile Lys Arg Asn Thr Pro Pro Gly Phe Gln Ala
                170                 175                 180
Ser Ser Phe Lys Thr Val Glu Val Asp Arg Lys Glu Gln Val Ala
                185                 190                 195
Leu Ile Met Asn Ser Ser Gly Ser Thr Gly Leu Pro Lys Gly Val
                200                 205                 210
Gln Leu Thr His Glu Asn Ile Val Thr Arg Phe Ser His Ala Arg
                215                 220                 225
Asp Pro Ile Tyr Gly Asn Gln Val Ser Pro Gly Thr Ala Val Leu
                230                 235                 240
 
Thr Val Val Pro Phe His His Gly Phe Gly Met Phe Thr Thr Leu
                245                 250                 255
Gly Tyr Leu Ile Cys Gly Phe Arg Val Val Met Leu Thr Lys Phe
                260                 265                 270
Asp Glu Glu Thr Phe Leu Lys Thr Leu Gln Asp Tyr Lys Cys Thr
                275                 280                 285
Ser Val Ile Leu Val Pro Thr Leu Phe Ala Ile Leu Asn Lys Ser
                290                 295                 300
Glu Leu Leu Asn Lys Tyr Asp Leu Ser Asn Leu Val Glu Ile Ala
                305                 310                 315
Ser Gly Gly Ala Pro Leu Ser Lys Glu Val Gly Glu Ala Val Ala
                320                 325                 330
Arg Arg Phe Asn Leu Pro Gly Val Arg Gln Gly Tyr Gly Leu Thr
                335                 340                 345
Glu Thr Thr Ser Ala Ile Ile Ile Thr Pro Glu Gly Asp Asp Lys
                350                 355                 360
Pro Gly Ala Ser Gly Lys Val Val Pro Leu Phe Lys Ala Lys Val
                365                 370                 375
Ile Asp Leu Asp Thr Lys Lys Ser Leu Gly Pro Asn Arg Arg Gly
                380                 385                 390
Glu Val Cys Val Lys Gly Pro Met Leu Met Lys Gly Tyr Val Asn
                395                 400                 405
Asn Pro Glu Ala Thr Lys Glu Leu Ile Asp Glu Glu Gly Trp Leu
                410                 415                 420
His Thr Gly Asp Ile Gly Tyr Tyr Asp Glu Glu Lys His Phe Phe
                425                 430                 435
Ile Val Asp Arg Leu Lys Ser Leu Ile Lys Tyr Lys Gly Tyr Gln
                440                 445                 450
Val Ala Pro Ala Glu Leu Glu Ser Ile Leu Leu Gln His Pro Asn
 
                455                 460                 465
Ile Phe Asp Ala Gly Val Ala Gly Leu Pro Asp Asp Asp Ala Gly
                470                 475                 480
Glu Leu Pro Ala Ala Val Val Val Leu Glu His Gly Lys Thr Met
                485                 490                 495
Thr Glu Lys Glu Ile Val Asp Tyr Val Ala Ser Gln Val Thr Thr
                500                 505                 510
Ala Lys Lys Leu Arg Gly Gly Val Val Phe Val Asp Glu Val Pro
                515                 520                 525
Lys Gly Leu Thr Gly Lys Leu Asp Ala Arg Lys Ile Arg Glu Ile
                530                 535                 540
Leu Ile Lys Ala Lys Lys Gly Gly Lys Ser Lys Leu
545 550 552
SEQ ID NO: 8
   SEQUENCE LENGTH: 1656
   SEQUENCE TYPE: nucleic acid
   STRANDEDNESS: single
   TOPOLOGY: linear
   MOLECULE TYPE: cDNA to mRNA
   ORIGINAL SOURCE: Luciola cruciata and Photinus pyralis
   SEQUENCE DESCRIPTION: 
ATG GAA AAC ATG GAA AAC GAT GAA AAT ATT GTA GTT GGA CCT AAA CCG 48
TTT TAC CCT ATC GAA GAG GGA TCT GCT GGA ACA CAA TTA CGC AAA TAC 96
ATG GAG CGA TAT GCA AAA CTT GGC GCA ATT GCT TTT ACA AAT GCA GTT 144
ACT GGT GTT GAT TAT TCT TAC GCC GAA TAC TTG GAG AAA TCA TGT TGT 192
CTA GGA AAA GCT TTG CAA AAT TAT GGT TTG GTT GTT GAT GGC AGA ATT 240
GCG TTA TGC AGT GAA AAC TGT GAA GAA TTT TTT ATT CCT GTA ATA GCC 288
GGA CTG TTT ATA GGT GTA GGT GTT GCA CCC ACT AAT GAG ATT TAC ACT 336
 
TTA CGT GAA CTG GTT CAC AGT TTA GGT ATC TCT AAA CCA ACA ATT GTA 384

TTT AGT TCT AAA AAA GGC TTA GAT AAA GTT ATA ACA GTA CAG AAA ACA 432

GTA ACT ACT ATT AAA ACC ATT GTT ATA CTA GAT AGC AAA GTT GAT TAT 480

CGA GGA TAT CAA TGT CTG GAC ACC TTT ATA AAA AGA AAC ACT CCA CCA 528

GGT TTT CAA GCA TCC AGT TTC AAA ACT GTG GAA GTT GAC CGT AAA GAA 576

CAA GTT GCT CTT ATA ATG AAC TCT TCG GGT TCT ACC GGT TTG CCA AAA 624

GGC GTA CAA CTT ACT CAC GAA AAT ATA GTC ACT AGA TTT TCT CAT GCT 672

AGA GAT CCG ATT TAT GGT AAC CAA GTT TCA CCA GGC ACC GCT GTT TTA 720

ACT GTC GTT CCA TTC CAT CAT GGT TTT GGT ATG TTC ACT ACT CTA GGG 768

TAT TTA ATT TGT GGT TTT CGT GTT GTA ATG TTA ACA AAA TTC GAT GAA 816

GAA ACA TTT TTA AAA ACT CTA CAA GAT TAT AAA TGT ACA AGT GTT ATT 864

CTT GTA CCG ACC TTG TTT GCA ATT CTC AAC AAA AGT GAA TTA CTC AAT 912

AAA TAC GAT TTG TCA AAT TTA GTT GAG ATT GCA TCT GGC GGA GCA CCT 960

TTA TCA AAA GAA GTT GGT GAA GCT GTT GCT AGA CGC TTT AAT CTT CCC 1008

GGT GTT CGT CAA GGT TAT GGT TTA ACA GAA ACA ACA TCT GCC ATT ATT 1056

ATT ACA CCG GAA GGT GAC GAT AAA CCA GGA GCT TCT GGA AAA GTC GTG 1104

CCG TTG TTT AAA GCA AAA GTT ATT GAT CTT GAT ACT AAA AAA TCT TTA 1152

GGT CCT AAC AGA CGT GGA GAA GTT TGT GTT AAA GGA CCT ATG CTT ATG 1200

AAA GGT TAT GTA AAT AAT CCA GAA GCA ACA AAA GAA CTT ATT GAC GAA 1248

GAA GGT TGG CTG CAC ACC GGA GAT ATT GGA TAT TAT GAT GAA GAA AAA 1296

CAT TTC TTT ATT GTC GAT CGT TTG AAG TCT TTA ATC AAA TAC AAA GGA 1344

TAT CAG GTG GCC CCC GCT GAA TTG GAA TCG ATA TTG TTA CAA CAC CCC 1392

AAC ATC TTC GAC GCG GGC GTG GCA GGT CTT CCC GAC GAT GAC GCC GGT 1440

GAA CTT CCC GCC GCC GTT GTT GTT TTG GAG CAC GGA AAG ACG ATG ACG 1488

GAA AAA GAG ATC GTG GAT TAC GTC GCC AGT CAA GTA ACA ACC GCG AAA 1536

AAG TTG CGC GGA GGA GTT GTG TTT GTG GAC GAA GTA CCG AAA GGT CTT 1584

ACC GGA AAA CTC GAC GCA AGA AAA ATC AGA GAG ATC CTC ATA AAG GCC 1632

AAG AAG GGC GGA AAG TCC AAA TTG 1656
SEQ ID NO: 9
   SEQUENCE LENGTH: 1656
   SEQUENCE TYPE: nucleic acid
   STRANDEDNESS: single
   TOPOLOGY: linear
   MOLECULE TYPE: cDNA to mRNA
   ORIGINAL SOURCE: Luciola lateralis, Photinus pyralis
   SEQUENCE DESCRIPTION: 
ATG GAA AAC ATG GAG AAC GAT GAA AAT ATT GTG TAT GGT CCT GAA CCA 48
TTT TAC CCT ATT GAA GAG GGA TCT GCT GGA GCA CAA TTG CGC AAG TAT 96
ATG GAT CGA TAT GCA AAA CTT GGA GCA ATT GCT TTT ACT AAC GCA CTT 144
ACC GGT GTC GAT TAT ACG TAC GCC GAA TAC TTA GAA AAA TCA TGC TGT 192
CTA GGA GAG GCT TTA AAG AAT TAT GGT TTG GTT GTT GAT GGA AGA ATT 240
GCG TTA TGC AGT GAA AAC TGT GAA GAA TTC TTT ATT CCT GTA TTA GCC 288
GGT TTA TTT ATA GGT GTC GGT GTG GCT CCA ACT AAT GAG ATT TAC ACT 336
CTA CGT GAA TTG GTT CAC AGT TTA GGC ATC TCT AAG CCA ACA ATT GTA 384
TTT AGT TCT AAA AAA GGA TTA GAT AAA GTT ATA ACT GTA CAA AAA ACG 432
GTA ACT GCT ATT AAA ACC ATT GTT ATA TTG GAC AGC AAA GTG GAT TAT 480
AGA GGT TAT CAA TCC ATG GAC AAC TTT ATT AAA AAA AAC ACT CCA CAA 528
GGT TTC AAA GGA TCA AGT TTT AAA ACT GTA GAA GTT AAC CGC AAA GAA 576
CAA GTT GCT CTT ATA ATG AAC TCT TCG GGT TCA ACC GGT TTG CCA AAA 624
GGT GTG CAA CTT ACT CAT GAA AAT TTG GTC ACT AGA TTT TCT CAC GCT 672
AGA GAT CCA ATT TAT GGA AAC CAA GTT TCA CCA GGC ACG GCT ATT TTA 720
ACT GTA GTA CCA TTC CAT CAT GGT TTT GGT ATG TTT ACT ACT TTA GGC 768
TAT CTA ACT TGT GGT TTT CGT ATT GTC ATG TTA ACG AAA TTT GAC GAA 816
GAG ACT TTT TTA AAA ACA CTG CAA GAT TAC AAA TGT TCA AGC GTT ATT 864
CTT GTA CCG ACT TTG TTT GCA ATT CTT AAT AGA AGT GAA TTA CTC GAT 912
AAA TAT GAT TTA TCA AAT TTA GTT GAA ATT GCA TCT GGC GGA GCA CCT 960
TTA TCT AAA GAA ATT GGT GAA GCT GTT GCT AGA CGT TTT AAT TTA CCG 1008
 
GGT GTT CGT CAA GGC TAT GGT TTA ACA GAA ACA ACC TCT GCA ATT ATT 1056

ATC ACA CCG GAA GGC GAT GAT AAA CCA GGT GCT TCT GGC AAA GTT GTG 1104

CCA TTA TTT AAA GCA AAA GTT ATC GAT CTT GAT ACT AAA AAA ACT TTG 1152

GGC CCG AAC AGA CGT GGA GAA GTT TGT GTA AAG GGT CCT ATG CTT ATG 1200

AAA GGT TAT GTA GAT AAT CCA GAA GCA ACA AGA GAA ATC ATA GAT GAA 1248

GAA GGT TGG TTG CAC ACA GGA GAT ATT GGG TAT TAC GAT GAA GAA AAA 1296

CAT TTC TTT ATC GTG GAT CGT TTG AAG TCT TTA ATC AAA TAC AAA GGA 1344

TAT CAG GTG GCC CCC GCT GAA TTG GAA TCG ATA TTG TTA CAA CAC CCC 1392

AAC ATC TTC GAC GCG GGC GTG GCA GGT CTT CCC GAC GAT GAC GCC GGT 1440

GAA CTT CCC GCC GCC GTT GTT GTT TTG GAG CAC GGA AAG ACG ATG ACG 1488

GAA AAA GAG ATC GTG GAT TAC GTC GCC AGT CAA GTA ACA ACC GCG AAA 1536

AAG TTG CGC GGA GGA GTT GTG TTT GTG GAC GAA GTA CCG AAA GGT CTT 1584

ACC GGA AAA CTC GAC GCA AGA AAA ATC AGA GAG ATC CTC ATA AAG GCC 1632

AAG AAG GGC GGA AAG TCC AAA TTG 1656
SEQ ID NO: 10
   SEQUENCE LENGTH: 552
   SEQUENCE TYPE: amino acid
   TOPOLOGY: linear
   MOLECULE TYPE: peptide
   ORIGINAL SOURCE: Luciola lateralis, Photinus pyralis
   SEQUENCE DESCRIPTION: 
Met Glu Asn Met Glu Asn Asp Glu Asn Ile Val Tyr Gly Pro Glu
1               5                   10                  15
Pro Phe Tyr Pro Ile Glu Glu Gly Ser Ala Gly Ala Gln Leu Arg
                20                  25                  30
Lys Tyr Met Asp Arg Tyr Ala Lys Leu Gly Ala Ile Ala Phe Thr
                35                  40                  45
Asn Ala Leu Thr Gly Val Asp Tyr Thr Tyr Ala Glu Tyr Leu Glu
 
                50                  55                  60
Lys Ser Cys Cys Leu Gly Glu Ala Leu Lys Asn Tyr Gly Leu Val
                65                  70                  75
Val Asp Gly Arg Ile Ala Leu Cys Ser Glu Asn Cys Glu Glu Phe
                80                  85                  90
Phe Ile Pro Val Leu Ala Gly Leu Phe Ile Gly Val Gly Val Ala
                95                  100                 105
Pro Thr Asn Glu Ile Tyr Thr Leu Arg Glu Leu Val His Ser Leu
                110                 115                 120
Gly Ile Ser Lys Pro Thr Ile Val Phe Ser Ser Lys Lys Gly Leu
                125                 130                 135
Asp Lys Val Ile Thr Val Gln Lys Thr Val Thr Ala Ile Lys Thr
                140                 145                 150
Ile Val Ile Leu Asp Ser Lys Val Asp Tyr Arg Gly Tyr Gln Ser
                155                 160                 165
Met Asp Asn Phe Ile Lys Lys Asn Thr Pro Gln Gly Phe Lys Gly
                170                 175                 180
Ser Ser Phe Lys Thr Val Glu Val Asn Arg Lys Glu Gln Val Ala
                185                 190                 195
Leu Ile Met Asn Ser Ser Gly Ser Thr Gly Leu Pro Lys Gly Val
                200                 205                 210
Gln Leu Thr His Glu Asn Leu Val Thr Arg Phe Ser His Ala Arg
                215                 220                 225
Asp Pro Ile Tyr Gly Asn Gln Val Ser Pro Gly Thr Ala Ile Leu
                230                 235                 240
Thr Val Val Pro Phe His His Gly Phe Gly Met Phe Thr Thr Leu
                245                 250                 255
Gly Tyr Leu Thr Cys Gly Phe Arg Ile Val Met Leu Thr Lys Phe
                260                 265                 270
 
Asp Glu Glu Thr Phe Leu Lys Thr Leu Gln Asp Tyr Lys Cys Ser
                275                 280                 285
Ser Val Ile Leu Val Pro Thr Leu Phe Ala Ile Leu Asn Arg Ser
                290                 295                 300
Glu Leu Leu Asp Lys Tyr Asp Leu Ser Asn Leu Val Glu Ile Ala
                305                 310                 315
Ser Gly Gly Ala Pro Leu Ser Lys Glu Ile Gly Glu Ala Val Ala
                320                 325                 330
Arg Arg Phe Asn Leu Pro Gly Val Arg Gln Gly Tyr Gly Leu Thr
                335                 340                 345
Glu Thr Thr Ser Ala Ile Ile Ile Thr Pro Glu Gly Asp Asp Lys
                350                 355                 360
Pro Gly Ala Ser Gly Lys Val Val Pro Leu Phe Lys Ala Lys Val
                365                 370                 375
Ile Asp Leu Asp Thr Lys Lys Thr Leu Gly Pro Asn Arg Arg Gly
                380                 385                 390
Glu Val Cys Val Lys Gly Pro Met Leu Met Lys Gly Tyr Val Asp
                395                 400                 405
Asn Pro Glu Ala Thr Arg Glu Ile Ile Asp Glu Glu Gly Trp Leu
                410                 415                 420
His Thr Gly Asp Ile Gly Tyr Tyr Asp Glu Glu Lys His Phe Phe
                425                 430                 435
Ile Val Asp Arg Leu Lys Ser Leu Ile Lys Tyr Lys Gly Tyr Gln
                440                 445                 450
Val Ala Pro Ala Glu Leu Glu Ser Ile Leu Leu Gln His Pro Asn
                455                 460                 465
Ile Phe Asp Ala Gly Val Ala Gly Leu Pro Asp Asp Asp Ala Gly
                470                 475                 480
Glu Leu Pro Ala Ala Val Val Val Leu Glu His Gly Lys Thr Met
 
                485                 490                 495
Thr Glu Lys Glu Ile Val Asp Tyr Val Ala Ser Gln Val Thr Thr
                500                 505                 510
Ala Lys Lys Leu Arg Gly Gly Val Val Phe Val Asp Glu Val Pro
                515                 520                 525
Lys Gly Leu Thr Gly Lys Leu Asp Ala Arg Lys Ile Arg Glu Ile
                530                 535                 540
Leu Ile Lys Ala Lys Lys Gly Gly Lys Ser Lys Leu
                545                 550
SEQ ID NO: 11
   SEQUENCE LENGTH: 21
   SEQUENCE TYPE: nucleic acid
   STRANDEDNESS: single
   TOPOLOGY:linear
   MOLECULE TYPE: other nucleic acid
   SEQUENCE DESCRIPTION:
   AGAGATCCAATTTATGGAAAC
SEQ ID NO: 12
   SEQUENCE LENGTH: 21
   SEQUENCE TYPE: nucleic acid
   STRANDEDNESS: single
   TOPOLOGY: linear
   MOLECULE TYPE: other nucleic acid
   SEQUENCE DESCRIPTION:
   AGCGTGAGAAAATCTGATCAC
SEQ ID NO: 13
   SEQUENCE LENGTH: 1644
   SEQUENCE TYPE: nucleic acid
   STRANDEDNESS: single
   TOPOLOGY: linear
   MOLECULE TYPE: cDNA to mRNA
   ORIGINAL SOURCE: Luciola lateralis
   SEQUENCE DESCRIPTION: 
ATG GAA AAC ATG GAG AAC GAT GAA AAT ATT GTG TAT GGT CCT GAA CCA 48
TTT TAC CCT ATT GAA GAG GGA TCT GCT GGA GCA CAA TTG CGC AAG TAT 96
ATG GAT CGA TAT GCA AAA CTT GGA GCA ATT GCT TTT ACT AAC GCA CTT 144
ACC GGT GTC GAT TAT ACG TAC GCC GAA TAC TTA GAA AAA TCA TGC TGT 192
CTA GGA GAG GCT TTA AAG AAT TAT GGT TTG GTT GTT GAT GGA AGA ATT 240
GCG TTA TGC AGT GAA AAC TGT GAA GAA TTC TTT ATT CCT GTA TTA GCC 288
GGT TTA TTT ATA GGT GTC GGT GTG GCT CCA ACT AAT GAG ATT TAC ACT 336
CTA CGT GAA TTG GTT CAC AGT TTA GGC ATC TCT AAG CCA ACA ATT GTA 384
TTT AGT TCT AAA AAA GGA TTA GAT AAA GTT ATA ACT GTA CAA AAA ACG 432
GTA ACT GCT ATT AAA ACC ATT GTT ATA TTG GAC AGC AAA GTG GAT TAT 480
AGA GGT TAT CAA TCC ATG GAC AAC TTT ATT AAA AAA AAC ACT CCA CAA 528
GGT TTC AAA GGA TCA AGT TTT AAA ACT GTA GAA GTT AAC CGC AAA GAA 576
CAA GTT GCT CTT ATA ATG AAC TCT TCG GGT TCA ACC GGT TTG CCA AAA 624
GGT GTG CAA CTT ACT CAT GAA AAT TTG GTG ATC AGA TTT TCT CAC GCT 672
AGA GAT CCA ATT TAT GGA AAC CAA GTT TCA CCA GGC ACG GCT ATT TTA 720
ACT GTA GTA CCA TTC CAT CAT GGT TTT GGT ATG TTT ACT ACT TTA GGC 768
TAT CTA ACT TGT GGT TTT CGT ATT GTC ATG TTA ACG AAA TTT GAC GAA 816
GAG ACT TTT TTA AAA ACA CTG CAA GAT TAC AAA TGT TCA AGC GTT ATT 864
CTT GTA CCG ACT TTG TTT GCA ATT CTT AAT AGA AGT GAA TTA CTC GAT 912
 
AAA TAT GAT TTA TCA AAT TTA GTT GAA ATT GCA TCT GGC GGA GCA CCT 960

TTA TCT AAA GAA ATT GGT GAA GCT GTT GCT AGA CGT TTT AAT TTA CCG 1008

GGT GTT CGT CAA GGC TAT GGT TTA ACA GAA ACA ACC TCT GCA ATT ATT 1056

ATC ACA CCG GAA GGC GAT GAT AAA CCA GGT GCT TCT GGC AAA GTT GTG 1104

CCA TTA TTT AAA GCA AAA GTT ATC GAT CTT GAT ACT AAA AAA ACT TTG 1152

GGC CCG AAC AGA CGT GGA GAA GTT TGT GTA AAG GGT CCT ATG CTT ATG 1200

AAA GGT TAT GTA GAT AAT CCA GAA GCA ACA AGA GAA ATC ATA GAT GAA 1248

GAA GGT TGG TTG CAC ACA GGA GAT ATT GGG TAT TAC GAT GAA GAA AAA 1296

CAT TTC TTT ATC GTG GAT CGT TTG AAG TCT TTA ATC AAA TAC AAA GGA 1344

TAT CAA GTA CCA CCT GCT GAA TTA GAA TCT GTT CTT TTG CAA CAT CCA 1392

AAT ATT TTT GAT GCC GGC GTT GCT GGC GTT CCA GAT CCT ATA GCT GGT 1440

GAG CTT CCG GGA GCT GTT GTT GTA CTT AAG AAA GGA AAA TCT ATG ACT 1488

GAA AAA GAA GTA ATG GAT TAC GTT GCT AGT CAA GTT TCA AAT GCA AAA 1536

CGT TTG CGT GGT GGT GTC CGT TTT GTG GAC GAA GTA CCT AAA GGT CTC 1584

ACT GGT AAA ATT GAC GGT AAA GCA ATT AGA GAA ATA CTG AAG AAA CCA 1632

GTT GCT AAG ATG 1644
SEQ ID NO: 14
   SEQUENCE LENGTH: 548
   SEQUENCE TYPE: amino acid
   TOPOLOGY: linear
   MOLECULE TYPE: peptide
   ORIGINAL SOURCE: Luciola lateralis
   SEQUENCE DESCRIPTION:

Met Glu Asn Met Glu Asn Asp Glu Asn Ile Val Tyr Gly Pro Glu
1               5                   10                  15
Pro Phe Tyr Pro Ile Glu Glu Gly Ser Ala Gly Ala Gln Leu Arg

                20                  25                  30
Lys Tyr Met Asp Arg Tyr Ala Lys Leu Gly Ala Ile Ala Phe Thr
                35                  40                  45
Asn Ala Leu Thr Gly Val Asp Tyr Thr Tyr Ala Glu Tyr Leu Glu
                50                  55                  60
Lys Ser Cys Cys Leu Gly Glu Ala Leu Lys Asn Tyr Gly Leu Val
                65                  70                  75
Val Asp Gly Arg Ile Ala Leu Cys Ser Glu Asn Cys Glu Glu Phe
                80                  85                  90
Phe Ile Pro Val Leu Ala Gly Leu Phe Ile Gly Val Gly Val Ala
                95                  100                 105
Pro Thr Asn Glu Ile Tyr Thr Leu Arg Glu Leu Val His Ser Leu
                110                 115                 120
Gly Ile Ser Lys Pro Thr Ile Val Phe Ser Ser Lys Lys Gly Leu
                125                 130                 135
Asp Lys Val Ile Thr Val Gln Lys Thr Val Thr Ala Ile Lys Thr
                140                 145                 150
Ile Val Ile Leu Asp Ser Lys Val Asp Tyr Arg Gly Tyr Gln Ser
                155                 160                 165
Met Asp Asn Phe Ile Lys Lys Asn Thr Pro Gln Gly Phe Lys Gly
                170                 175                 180
Ser Ser Phe Lys Thr Val Glu Val Asn Arg Lys Glu Gln Val Ala
                185                 190                 195
Leu Ile Met Asn Ser Ser Gly Ser Thr Gly Leu Pro Lys Gly Val
                200                 205                 210
Gln Leu Thr His Glu Asn Leu Val Ile Arg Phe Ser His Ala Arg
                215                 220                 225

Asp Pro Ile Tyr Gly Asn Gln Val Ser Pro Gly Thr Ala Ile Leu
                230                 235                 240
Thr Val Val Pro Phe His His Gly Phe Gly Met Phe Thr Thr Leu
                245                 250                 255
Gly Tyr Leu Thr Cys Gly Phe Arg Ile Val Met Leu Thr Lys Phe
                260                 265                 270
Asp Glu Glu Thr Phe Leu Lys Thr Leu Gln Asp Tyr Lys Cys Ser
                275                 280                 285
Ser Val Ile Leu Val Pro Thr Leu Phe Ala Ile Leu Asn Arg Ser
                290                 295                 300
Glu Leu Leu Asp Lys Tyr Asp Leu Ser Asn Leu Val Glu Ile Ala
                305                 310                 315
Ser Gly Gly Ala Pro Leu Ser Lys Glu Ile Gly Glu Ala Val Ala
                320                 325                 330
Arg Arg Phe Asn Leu Pro Gly Val Arg Gln Gly Tyr Gly Leu Thr
                335                 340                 345
Glu Thr Thr Ser Ala Ile Ile Ile Thr Pro Glu Gly Asp Asp Lys
                350                 355                 360
Pro Gly Ala Ser Gly Lys Val Val Pro Leu Phe Lys Ala Lys Val
                365                 370                 375
Ile Asp Leu Asp Thr Lys Lys Thr Leu Gly Pro Asn Arg Arg Gly
                380                 385                 390
Glu Val Cys Val Lys Gly Pro Met Leu Met Lys Gly Tyr Val Asp
                395                 400                 405
Asn Pro Glu Ala Thr Arg Glu Ile Ile Asp Glu Glu Gly Trp Leu
                410                 415                 420
His Thr Gly Asp Ile Gly Tyr Tyr Asp Glu Glu Lys His Phe Phe

                425                 430                 435
Ile Val Asp Arg Leu Lys Ser Leu Ile Lys Tyr Lys Gly Tyr Gln
                440                 445                 450
Val Pro Pro Ala Glu Leu Glu Ser Val Leu Leu Gln His Pro Asn
                455                 460                 465
Ile Phe Asp Ala Gly Val Ala Gly Val Pro Asp Pro Ile Ala Gly
                470                 475                 480
Glu Leu Pro Gly Ala Val Val Val Leu Lys Lys Gly Lys Ser Met
                485                 490                 495
Thr Glu Lys Glu Val Met Asp Tyr Val Ala Ser Gln Val Ser Asn
                500                 505                 510
Ala Lys Arg Leu Arg Gly Gly Val Arg Phe Val Asp Glu Val Pro
                515                 520                 525
Lys Gly Leu Thr Gly Lys Ile Asp Gly Lys Ala Ile Arg Glu Ile
                530                 535                 540
Leu Lys Lys Pro Val Ala Lys Met
                545

The following is described herein:
1. A bioluminescent protein with improvements in catalytic efficiency or stability.
2. A bioluminescent protein according to paragraph 1 wherein the improvements include at least one of 5 kinds of improvements in substrate specificity and maximum reaction rate in respect of catalytic efficiency, and thermal stability, pH stability and stability at low ion concentration in respect of stability.
3. A bioluminescent protein according to paragraph 1 or paragraph 2, which is a luciferase derived from beetles (Coleoptera).
4. A bioluminescent protein according to paragraph 1 or paragraph 2 which is a luciferase derived from fireflies.
5. A process for producing the mutant-type bioluminescent protein of paragraph 1 or paragraph 2 which comprises modifications to a bioluminescent protein precursor.
6. A process for producing the mutant-type bioluminescent protein of paragraph 1 or paragraph 2 wherein said modifications involve the replacement, alteration, removal and addition of at least one amino acid and the fusion of a plurality of proteins.
7. A bioluminescent protein according to paragraph 1 or paragraph 2 which has firefly luciferase activity and comprises a plurality of firefly luciferases fused therein.
8. A bioluminescent protein according to paragraph 1 or paragraph 2, which has firefly-luciferase activity and comprises luciferases from Genji firefly (Luciola cruciata) and American firefly (Photinus pyralis) fused therein.
9. A bioluminescent protein according to paragraph 1 or paragraph 2 which has firefly luciferase activity and comprises luciferases from Heike firefly (Luciola lateralis) and American firefly (Photinus pyralis) fused therein.
10. A bioluminescent protein according to paragraph 1 or paragraph 2 which has firefly luciferase activity and comprises luciferases from Genji firefly (Luciola cruciata) and Heike firefly (Luciola lateralis) fused therein.
11. A bioluminescent protein according to paragraph 1 or paragraph 2 which has firefly luciferase activity and has a mutation in an amino acid residue corresponding to the 219-position of the Luciola cruciata luciferase.
12. A bioluminescent protein according to paragraph 1 or paragraph 2 which has firefly luciferase activity and has a mutation in an amino acid residue corresponding to the 239-position of the Luciola cruciata luciferase.
13. A bioluminescent protein according to paragraph 1 or paragraph 2 which has firefly luciferase activity and has a mutation in an amino acid residue corresponding to the 290-position of the Luciola cruciata luciferase.
14. A bioluminescent protein according to paragraph 7 which comprises an N-terminal region of a first firefly luciferase and a C-terminal region of a second firefly luciferase.
15. A bioluminescent protein according to paragraph 8 having the sequence of SEQ ID NO: 5 or SEQ ID NO: 7, or a mutant thereof which comprises a substitution at a residue corresponding to positions 219, 239 or 290 of Luciola cruciata luciferase.
16. A bioluminescent protein according to paragraph 9 having the sequence of SEQ ID NO: 10 or a mutant thereof which comprises a substitution at a residue corresponding to positions 219, 239 or 290 of Luciola cruciata luciferase.

## Claims

1. A bioluminescent protein having the sequence of SEQ ID NO: 5, which has firefly-luciferase activity and comprises luciferases from Genji firefly (Luciola cruciata) and American firefly (Photinus pyralis) fused therein.

2. A bioluminescent protein having the sequence of SEQ ID NO: 7, which has firefly-luciferase activity and comprises luciferases from Genji firefly (Luciola cruciata) and American firefly (Photinus pyralis) fused therein.

3. A bioluminescent protein according to claim 2 which has firefly luciferase activity and has a mutation in an amino acid residue corresponding to the 219-position of the Genji firefly (Luciola cruciata) luciferase.

4. A bioluminescent protein according to claim 2 which has firefly luciferase activity and has a mutation in an amino acid residue corresponding to the 239-position of the Genji firefly (Luciola cruciata) luciferase.

5. A bioluminescent protein according to claim 2 which has firefly luciferase activity and has a mutation in an amino acid residue corresponding to the 290-position of the Genji firefly (Luciola cruciata) luciferase.

6. A bioluminescent protein having the sequence of SEQ ID NO: 10 which has firefly luciferase activity and comprises luciferases from Heike firefly (Luciola lateralis) and American firefly (Photinus pyralis) fused therein.

## Patentansprüche

1. Biolumineszierendes Protein mit der Sequenz von Seq.-ID Nr. 5, das Leuchtkäfer-Luciferase-Aktivität aufweist und darin fusionierte Luciferasen aus dem Genji-Leuchtkäfer (Luciola cruciata) und der amerikanischen Feuerfliege (Photinus pyralis) umfasst.

2. Biolumineszierendes Protein mit der Sequenz von Seq.-ID Nr. 7, das Leuchtkäfer-Luciferase-Aktivität aufweist und darin fusionierte Luciferasen aus dem Genji-Leuchtkäfer (Luciola cruciata) und der amerikanischen Feuerfliege (Photinus pyralis) umfasst.

3. Biolumineszierendes Protein nach Anspruch 2, das Leuchtkäfer-Luciferase-Aktivität und eine Mutation an einem der 219-Position der Luciferase des Genji-Leuchtkäfers (Luciola cruciata) entsprechenden Aminosäurerest aufweist.

4. Biolumineszierendes Protein nach Anspruch 2, das Leuchtkäfer-Luciferase-Aktivität und eine Mutation an einem der 239-Position der Luciferase des Genji-Leuchtkäfers (Luciola cruciata) entsprechenden Aminosäurerest aufweist.

5. Biolumineszierendes Protein nach Anspruch 2, das Leuchtkäfer-Luciferase-Aktivität und eine Mutation an einem der 290-Position der Luciferase des Genji-Leuchtkäfers (Luciola cruciata) entsprechenden Aminosäurerest aufweist.

6. Biolumineszierendes Protein mit der Sequenz von Seq.-ID Nr. 10, das Leuchtkäfer-Luciferase-Aktivität aufweist und darin fusionierte Luciferasen aus dem Heike-Leuchtkäfer (Luciola lateralis) und der amerikanischen Feuerfliege (Photinus pyralis) umfasst.

## Revendications

1. Protéine bioluminescente ayant la séquence de SEQ ID NO: 5, qui a une activité de luciférase de luciole et comprend des luciférases de la luciole Genji (Luciola cruciata) et de la luciole Américaine (Photinus pyralis) fondue dans celle-ci.

2. Protéine bioluminescente ayant la séquence de SEQ ID NO: 7, qui a une activité de luciférase de luciole et comprend des luciférases de la luciole Genji (Luciola cruciata) et de la luciole Américaine (Photinus pyralis) fondue dans celle-ci.

3. Protéine bioluminescente selon la revendication 2, qui a une activité de luciférase de luciole et a une mutation dans un résidu d'acide aminé correspondant à la position 219 de la luciférase de la luciole Genji (Luciola cruciata).

4. Protéine bioluminescente selon la revendication 2, qui a une activité de luciférase de luciole et a une mutation dans un résidu d'acide aminé correspondant à la position 239 de la luciférase de la luciole Genji (Luciola cruciata).

5. Protéine bioluminescente selon la revendication 2, qui a une activité de luciférase de luciole et a une mutation dans un résidu d'acide aminé correspondant à la position 290 de la luciférase de la luciole Genji (Luciola cruciata).

6. Protéine bioluminescente ayant la séquence de SEQ ID NO: 10, qui a une activité de luciférase de luciole et comprend des luciférases de la luciole Heike (Luciola lateralis) et de la luciole Américaine (Photinus pyralis) fondue dans celle-ci.
